# EUROPEAN PATENT APPLICATION

(11) **EP 4 840 965 A1**
(43) Date of publication of application: **18.11.2026**
(21) Application number: 26178521.6
(22) Date of filing: 13.05.2026
(51) Int. Cl.: C12N 15/86, C12N 15/69, C12N 15/70

(54) **MINICIRCLES WITH FULL-LENGTH INVERTED REPEATS AND METHODS FOR PRODUCING THE SAME**

(30) Priority: 13.05.2025 EP 25176024
(71) Applicant: PlasmidFactory GmbH, 33607 Bielefeld (DE)
(72) Inventor: Schleef, Martin, 33739 Bielefeld (DE); Shankar, Ram, 33649 Bielefeld (DE); Schäffer, Nina, 32130 Enger (DE); Müller, Kristian Mark, 33615 Bielefeld (DE); Radukic, Marco, 33615 Bielefeld (DE)
(74) Representative: Grund, Martin

(57) **Abstract**

The present invention relates to a method for propagating plasmids and minicircles containing at least one inverted repeat in *Escherichia coli,* the method comprising the step of culturing the *E. coli* bacteria at a temperature of at least 39°C. Furthermore, the invention relates to a plasmid or a minicircle comprising a cargo nucleic acid comprising at least one inverted repeat obtainable by a method of the present invention. The invention further relates to a cargo nucleic acid comprising at least one inverted repeat obtainable by a method of the present invention.

## Description

### FIELD OF THE INVENTION

The invention lies in the field of plasmid and minicircle technology and relates to the propagation of plasmids and minicircles as transfer vectors in *E. coli* bacteria. The plasmids and minicircles comprise at least one inverted repeat that is maintained by the methods of the invention.

### BACKGROUND

Methods for propagating plasmids in *Escherichia coli* bacteria are well known. In particular, maintaining the length and integrity of inverted repeat (IR) sequences presents a challenge that has not been fully addressed by previous approaches.

Nucleic acids containing one or more inverted repeats, optionally as palindromic sequences, have a wide range of uses in research and therapy. For instance, inverted terminal repeats (ITRs) are regularly found. Nucleic acids can be genetic encoding material that allow expression of polypeptides by the host cell or that can be intended for other purposes such as for decreasing expression rates of inherent genes of the target cell (e.g., by encoding for silencing RNA (siRNA) or micro RNA, etc.). The presence of one or more foreign nucleic acids (also: (deoxy)ribonucleic acids) in the target cell can be transient or persistent. It may be inserted into the target cell's genome or may remain extrachromosomal. For instance, viral nucleic acids such as nucleic acids originating from adeno-associated virus (AAV), commonly used as vectors to import one or more cargo sequences such as one or more foreign nucleic acids (e.g., DNA and, in particular in other viruses, RNA) into eukaryotic cells, are known to carry inverted terminal repeats (ITRs). Vectors such as viral vectors allow a systemic and cell-type specific import of cargo sequences *in vivo*. This also allows gene therapy of humans and other mammals.

When using nucleic acids on a large scale, as required for therapeutic uses, efficient preparation thereof is required. Technical means such as polymerase chain reaction (PCR) often do not provide sufficient amounts with reasonable effort or are inhibited by the inverted repeat structure. Therefore, when higher amounts of nucleic acids are desired, often plasmids containing the nucleic acid sequence (also: cargo nucleic acid) of interest are amplified in *Escherichia coli* (*E. coli*) bacteria. Such propagation of plasmids efficiently provides a reasonably large amount of plasmid DNA. Propagation of *E. coli* bacteria containing the plasmids may be performed as a seed train for several consecutive days to reach sufficient biomass for large-scale plasmid production in a stirred tank reactor.

However, when preparing cargo nucleic acids that include at least one inverted repeat, in particular, at least one inverted repeat of longer nucleic acid sequence length, such as an ITR, there are special challenges when propagating the plasmids. Doherty *et al.* (4), for instance, reported that it is challenging to obtain libraries of lambda phages that comprise such palindromic sequences in commonly used *E. coli* strains. Often, deletions of inverted repeats occur when preparing plasmids comprising ITRs, as shown in Samulski *et al.* (14). Deletion mutants have also been purposefully prepared by Samulski *et al.* (15), of which some were considered as not suitable for replication as taught in Samulski *et al.* (16). Indeed, deletions can significantly impair the quality of the inverted repeats of viral nucleic acids, as also taught by Wang *et al.* (20). Analysis of the deletions in a production process can be laborious and challenging (Radukic *et al.*; 11).

It is desirable to maintain the integrity of the inverted repeats in the final product, as this may support the functionality of the desired nucleic acid such as, e.g., a viral nucleic acid which may be used for insertion into the target cell's genome and/or for replication.

Unfortunately, deletions of sequence motifs forming extensive intra- and/or inter-sequence secondary structures, such as inverted repeats, are found, for example, when propagating plasmids that contain an ITR in *E. coli* bacteria by routine techniques.

Typically, *E. coli* bacteria are cultivated at a temperature of 37°C. Propagation of plasmids is typically performed by means of cultivating the proliferating *E. coli* bacteria at such standard conditions. When the expression of labile proteins is intended, the temperature may be decreased to 30°C or even ambient temperature. Decreasing the temperature may reduce the proliferation of the bacteria and may have other effects reducing the plasmid copy number and may thus lead to long processing times and decreased yields per time. Additionally, undesirable deletions of inverted repeats was observed at such conditions. Also, applying a brief heat shock, as known in the art, did not affect the quality of the inverted repeats of a viral nucleic acid.

Remaut *et al.* (12) describe a brief temperature shift from 28°C to 42°C of 3 or 6 h to select plasmids with temperature-inducible promoters. In another instance, a plasmid containing a temperature-inducible promoter was maintained at 30°C before shifting the culture to 42°C for 16 h to induce the expression of a protein of interest (Waldman *et al.*; 19). Furthermore, brief temperature shocks of less than two minutes have been described. These methods, however, do not allow sufficient preparation of cargo nucleic acids that include at least one inverted repeat and maintain the integrity of the inverted repeat.

Various conditions of cultivating *E. coli* bacteria and propagating plasmids comprising at least one inverted repeat were previously tested, such as using recombination-deficient *E. coli* strains, selecting smaller colonies, and reducing culture times. The undesirable deletion of inverted repeats has nonetheless remained a challenging issue, as described in Wilmott *et al.* (21). An undesirable heterogeneity of more and less deleted versions may be the result of the standard processes known in the art. Some deletions are detectable by using digestion enzymes. For others, a more detailed analysis is required.

Production processes try to avoid the problem of undesirable deletions by truncating the inverted repeats, like ITRs, from the outset, such as by 22 or 11 base pairs in the ITR internal inverted repeat, in addition to truncating the ITR outer inverted repeat by 15 base pairs. A meta-analysis of published plasmids available for AAV research shows that all available ITRs are truncated at least by 15 base pairs (cf. Example 1). This is, however, not desirable. On the one hand, the disadvantage remains that deletions still occur. On the other hand, the efficacy of nucleic acids, such as viral nucleic acids to be used in the production of vectors such as viral vectors, may be negatively affected.

Therefore, there is an unmet need for an efficient method for propagating plasmids containing at least one inverted repeat that widely maintains the integrity the inverted repeat.

### SUMMARY OF THE INVENTION

The present invention therefore relates to modified methods for cultivating *E. coli* bacteria containing at least one plasmid, wherein the plasmid comprises at least one IR sequence, while preserving the integrity and length of the IR sequence as much as possible. In particular, cultivation of the bacteria at elevated temperature and the use of selected bacterial genotypes makes it possible to obtain full-length inverted terminal repeats (ITRs) on plasmids.

The present invention relates in particular to the cultivation of such a plasmid at an industrial scale with the aim of producing the plasmid in a higher quantity and a defined quality in the context of industrial plasmid DNA production for use in adeno-associated virus (AAV) vector production.

Furthermore, the invention relates to the provision of the transfer vector in the form of a minicircle and associated methods for propagating *E. coli* bacteria comprising the minicircles or parental plasmids, wherein the IRs contained on the minicircles are retained in as full a length as possible.

Compared to providing the transfer vector as a plasmid, minicircles possess certain desirable properties. In particular, minicircles lack prokaryotic backbone sequences, thus the resulting supercoiled constructs consist exclusively of the gene of interest (GOI) and elements related to and relevant for use in eukaryotic target cells. For example, in the case of AAV vector production, a transfer minicircle comprises only the gene of interest flanked by the ITR sequences and a short scar sequence.

One of the immediate advantages of a minicircle is the increased transfection efficiency due to its smaller size. A previous study demonstrated that the use of minicircles for the transfer, replication, and packaging functions during AAV vector production results in vectors that are essentially free of mispackaging events, whereas the use of plasmids leads to undesirable mispackaging of bacterial backbone sequence elements in the recombinant vector particles, as described by Schnödt *et al.* (17). Thus, the use of minicircles has enabled tremendous improvement in the genetic purity of the vectors. The absence of prokaryotic motifs arising from the plasmid backbone such as unmethylated CpG motifs is expected to reduce the risk of induction of an immune response against the vector upon application to target cells. The possibility of epigenetic silencing of the recombinant vector is also expected to be reduced. Other studies have also suggested the possibility of increased vector genome titer when using minicircle transfer constructs as opposed to plasmids.

These advantages, in conjunction with the advantages of maintaining full-length ITR sequences, namely increased genome titer, genetic purity and successful packaging, as described by Radukic *et al.* (11), led to the inventive development of a transfer minicircle containing full-length ITR sequences and methods for propagating and providing the same.

Thus, in a first aspect, the invention relates to a minicircle comprising at least one inverted repeat of viral origin, wherein said inverted repeat is essentially not truncated.

In one embodiment, said inverted repeat has a sequence identity of at least 90%, at least 92%, or at least 95% compared to the corresponding wild-type inverted repeat of the virus of origin.

In one embodiment, said inverted repeat is an inverted terminal repeat (ITR), preferably an ITR of an adeno-associated virus (AAV).

In one embodiment, said inverted repeat is at least 100 bp, at least 110 bp, at least 120 bp, at least 130 bp, at least 140 bp, or at least 150 bp in length.

In a second aspect, the invention relates to a composition comprising a population of minicircles as described above, wherein the inverted repeats of the population of minicircles have an average sequence identity of at least 90%, at least 92%, or at least 95% to the corresponding wild-type inverted repeat of the virus of origin.

In a third aspect, the invention relates to a method for producing a minicircle from a parental plasmid, wherein the minircle comprises at least one inverted repeat, the method comprising the steps:
(i) transforming *Escherichia coli* (*E. coli*) cells with said parental plasmid;
(ii) continuously cultivating said *E. coli* cells at a temperature of at least 39°C, comprising propagating the parental plasmid of step (i); and
(iii) inducing the formation of minicircles from the propagated parental plasmid of step (ii).

In one embodiment, cultivating in step (ii) is performed at 42°C.

In one embodiment, cultivating in step (ii) is performed for at least 8 hours, preferably for at least 16 hours.

In one embodiment, step (iii) is performed for at least 30 minutes, at least 1 hour, preferably for 1-4 hours or 4-8 hours.

In one embodiment, the method further comprising the step:
(iv) isolating the minicircles.

In one embodiment, said inverted repeat is essentially not truncated, preferably wherein said inverted repeat has a sequence identity of at least 90%, at least 92%, or at least 95% compared to the corresponding inverted repeat on the parental plasmid.

In one embodiment, the inverted repeats of the of the minicircles obtained in step (iii) have an average sequence identity of at least 90%, at least 92%, or at least 95% to the corresponding inverted repeat of the parental plasmid.

In one embodiment, the inverted repeat is of viral origin.

In one embodiment, said inverted repeat is an ITR, preferably an ITR of an AAV.

In one embodiment, said at least one ITR comprises an ITR proximal to the origin of replication of the parental plasmid (origin proximal ITR) and an ITR distal to the origin of replication of the parental plasmid (origin distal ITR).

In one embodiment, said inverted repeat is at least 100 bp, at least 110 bp, at least 120 bp, at least 130 bp, at least 140 bp, or at least 150 bp in length.

In one embodiment, the *E. coli* cells are wild-type *E. coli* cells, preferably K12 or B derivatives of *E. coli*.

In one embodiment, the *E. coli* cells have the genotype sbcC⁻ or sbcD⁻.

In one embodiment, the *E. coli* cells comprise a chromosomal recA⁻ and/or endA⁻ mutation.

In one embodiment, the minicircle comprises at least one sequence encoding:
one or more polypeptides;
RNA; or
DNA;
preferably wherein the at least one sequence has therapeutic utility.

In a fourth aspect, the invention relates to a method of propagating a plasmid in *E. coli* cells, wherein the plasmid comprises at least one inverted repeat, the method comprising the steps:
(i) transforming *E. coli* cells with said plasmid;
(ii) continuously cultivating the *E. coli* cells comprising the plasmid of step (i) in a pre-culture at a temperature of at least 39°C for at least 8 hours, preferably for at least 16 hours; and
(iii) inoculating a main culture with *E.* coli cells from the pre-culture of step (ii) and cultivating the main culture at a temperature of at least 39°C.

In one embodiment, the main culture has a volume of at least 5 l, at least 10 l, at least 20 l, at least 30 l, at least 50 l, at least 75 l, at least 100 l, at least 200 l, at least 300 l, at least 500 l, or at least 1000 l.

In one embodiment, after 3 days of cultivation in step (ii) and step (iii), at least 80%, at least 90%, or at least 92% of the inverted repeats of the plasmids in the main culture of step (iii) are essentially not truncated.

In one embodiment, after 3 days of cultivation in step (ii) and step (iii), the inverted repeats of the plasmids in the main culture of step (iii) have an average sequence identity of at least 90%, at least 92%, or at least 95% compared to the inverted repeat of the plasmid of step (i).

In one embodiment, the inverted repeat is of viral origin.

In one embodiment, said inverted repeat is an ITR, preferably an ITR of an AAV.

In one embodiment, said at least one ITR comprises an ITR proximal to the origin of replication of the plasmid (origin proximal ITR) and an ITR distal to the origin of replication of the plasmid (origin distal ITR).

In one embodiment, said inverted repeat is at least 100 bp, at least 110 bp, at least 120 bp, at least 130 bp, at least 140 bp, or at least 150 bp in length.

In one embodiment, the *E. coli* cells are wild-type *E. coli* cells, preferably K12 or B derivatives of *E. coli*.

In one embodiment, the *E. coli* cells have the genotype sbcC⁻ or sbcD⁻.

In one embodiment, the *E. coli* cells comprise a chromosomal recA⁻ and/or endA⁻ mutation.

In one embodiment, the plasmid comprises at least one sequence encoding:
one or more polypeptides;
RNA; or
DNA;
preferably wherein the at least one sequence has therapeutic utility.

In one embodiment, said plasmid is a parental plasmid for the production of minicircles comprising at least one inverted repeat.

In one embodiment, the method further comprises the step:
(iv) inducing the formation of minicircles from said parental plasmid in the main culture of step (iii);
wherein step (iv) is performed for at least 30 minutes, at least 1 hour, preferably for 1-4 hours or 4-8 hours.

In one embodiment, the method further comprising the step:
(iv) isolating the minicircles.

### BRIEF DESCRIPTION OF THE DRAWINGS

**Fig. 1**: Analysis of the proximal and distal ITR length distribution of plasmid preparations from three stages of biomass harvest after bioreactor cultivation of full-length ITR plasmid pZMB0990 in industrially relevant *E. coli* production strain PF03 (carrying a mutation in the sbcC gene) at a consistently elevated temperature of 42°C.
**Fig. 2**: Scheme of the production of minicircle MC3265 from parental plasmid PF3265PP_O1. The parental plasmid includes the ITRs and intervening sequences from plasmid pZMB0990.

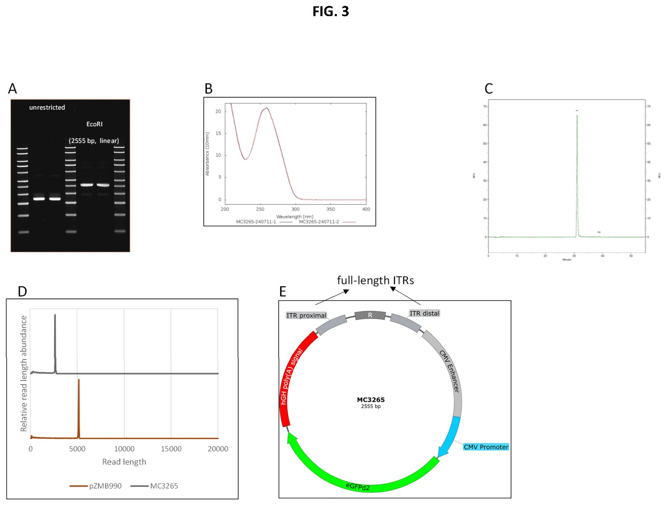

**Fig. 3**: Quality control (QC) data for the purified minicircle MC3265 with full-length ITRs. (A) Agarose gel electrophoresis analysis showing the size of the minicircle; (B) spectrophotometric analysis using a wavelength scan between 200 nm and 400 nm to measure purity and concentration; (C) capillary gel electrophoresis showing the minicircle in supercoiled conformation (99.8%); (D) NGS analysis showing a high frequency of the monomeric conformation; and (E) schematic map of MC3265 showing the full-length ITRs, the CMV promoter, the eGFP reporter gene, and the hGH poly(A) signal sequences.
**Fig. 4**: Analysis of the ITR length distribution in the initial plasmid pZMB0990 and the resulting minicircle MC3265. The initial plasmid pZMB0990 showed a deviation of 8% from the original full length of the ITR, while the minicircle MC3265 showed a deviation of only 4%.
**Fig. 5**: (A) Comparison of reporter gene expression using plasmid or minicircle transfer vectors; (B) relative vector genome and capsid yields using plasmid and minicircle transfer vectors.
**Fig. 6:** (A) Comparison of vector genome yield; (B) Rate of mispackaging events and (C) capsid loading when using plasmid or minicircle transfer vectors on the basis of equal mass of total DNA transfected.
**Fig. 7:** (A) ITR length distribution analysis following Next-Generation Sequencing of various ITR containing plasmids. Starting from a plasmid containing a functional but truncated ITR (BC10), two derivatives with full-length ITRs (wildtype length), containing two different backbone sequences - ampicillin resistance in BC04-06 and kanamycin resistance in BC07-09, were cloned. (B) Final ITR length characterization of the newly generated constructs.

### DETAILED DESCRIPTION OF THE INVENTION

### Minicircles

In one aspect, the invention relates to a minicircle comprising at least one inverted repeat of viral origin, wherein said inverted repeat is essentially not truncated.

As used herein, "minicircle" refers to a circular double-stranded DNA containing at least one cargo nucleic acid and being essentially free of prokaryotic nucleic acid sequences typically found in plasmids, such as origins of replication, marker genes, or resistance genes. In particular, minicircles contain only the gene of interest (GOI) intended for application in the target eukaryotic cell or tissue, promoters that serve to express gene(s) contained in said expression cassette (if any) and other associated elements for eukaryotic gene expression such as a polyadenylation signal sequence or even eukaryotic selection markers or gene sequences that have a regulatory function in the target eukaryotic cell, in addition to a short scar sequence resulting from their production. This makes minicircles particularly suitable for introducing desired nucleic acid sequences into target cells, since their relative smaller size increases electroporation efficiency in non-viral approaches as well as allowing for a reduction in the required amount of DNA for modification of the eukaryotic cells and thereby avoiding DNA-related toxicity. In case of viral vector production, removal of the prokaryotic backbone sequences contributes significantly to an increase in genomic purity by drastically reducing the chances of such sequences being falsely packaged in vector particles. The viral vector preparation is thereby rendered less immunogenic and less susceptible to epigenetic silencing.

Preferably, the minicircles of the invention are supercoiled.

As used herein, the term "inverted repeat" (IR) may be understood as commonly understood in the art in the broadest sense. Briefly, an inverted repeat may be understood as a nucleic acid comprising a respective sequence of nucleotides that is followed downstream by its reverse complement on the same strand. These complementary sequences may or may not be interrupted by other sequences, which may themselves be other inverted repeats. When there is no intervening sequence, the composite sequence may be understood as a palindromic sequence.

Inverted repeats are sometimes called hairpin structures, hairpin loops, or stem-loop structures. Inverted repeats present on double-stranded DNA may form structures that resemble holiday junctions, sometimes called cruciforms.

In the minicircles of the invention, the inverted repeat is of viral origin. In a preferred embodiment, the inverted repeat is of adeno-associated virus (AAV) origin, in particular from AAV serotype 2. Inverted repeats of AAV are exemplarily described in Lusby *et al.* (9), Berns (3), and Wilmott *et al.* (21). In a preferred embodiment, the cargo nucleic acid is selected from the group consisting of recombinant AAV vector genomes. In a preferred embodiment, the cargo nucleic acid is a recombinant AAV vector genome optionally comprising one or more cargo peptide and/or protein expression cassettes.

In a preferred embodiment, the at least one inverted repeat is an inverted terminal repeat (ITR). The term "inverted terminal repeat" is used herein as commonly used in the art in the broadest sense. Briefly, an ITR may be understood as an inverted repeat located at a terminus of a DNA sequence of interest.

In the minicircles of the invention, the at least one ITR is an ITR of viral origin, typically referred to as a wild-type ITR. In a preferred embodiment, the at least one ITR is an ITR of an AAV serotype 2, which can also be designated as AAV2-ITR. AAV2-ITRs are exemplarily described in Lusby *et al.* (9), Berns (3), and Wilmott *et al.* (21). Many other AAV-ITRs from other AAV serotypes are known in the art. In addition, synthetic AAV-ITRs with altered sequences are known in the art.

AAV-ITRs are characterized by having four sequence elements, typically referred to as A, B, C, and D, arranged in a pattern of A-B-B'-C-C'-A'-D, where the prime indicates the reverse complement of the respective sequence element. The pattern A-B-B'-C-C'-A'-D is typically referred to as the FLIP-configuration, and A-C-C'-B-B'-A'-D is typically referred to as the FLOP configuration. The D sequence is not part of an inverted repeat but is typically still considered part of the ITR. The person skilled in the art is aware that any combination of the sequence elements A, B, C, and D able to form an inverted repeat would still be considered an ITR. For example, the pattern A-A'-D would still be considered an ITR. The person skilled in the art is aware that any combination of shortened sequence elements able to form an inverted repeat would still be considered an ITR. For example, the pattern A(short)-A'-D would still be considered an ITR when A(short) was shorter in length compared to A'.

In a preferred embodiment, the inverted repeat is characterized in that it is at least 20 bp in length, at least 10 bp in length, at least 25 bp in length, at least 50 bp in length, at least 75 bp in length, at least 80 bp in length, at least 90 bp in length, at least 93 bp in length, at least 94 bp in length, at least 100 bp in length, at least 110 bp in length, at least 120 bp in length, at least 130 bp in length, or at least 140 bp in length, or at least 150 bp in length.

In a preferred embodiment, the inverted repeat is characterized in that it is not more than 1000 bp in length, not more than 300 bp in length, not more than 200 bp in length, not more than 150 bp in length, not more than 100 bp in length, or not more than 50 bp in length.

The molar content of guanosine and cytidine (guanosine/cytidine content, GC content), based on the overall nucleoside content, of the inverted repeat may be any content. In a preferred embodiment, the inverted repeat is characterized in that it has a guanosine/cytidine content of at least 50 mol%, at least 60 mol%, at least 65 mol%, at least 70 mol%, at least 75 mol%, or at least 80 mol%, based on the overall nucleoside content.

In a preferred embodiment, the inverted repeat is characterized in that:
it is of viral origin, in particular AAV origin;
it is an ITR, in particular an ITR of AAV origin;
it is at least 131 base pairs in length including the D-sequence; and/or
it has a guanosine/cytidine content of at least 70 mol%, based on the overall nucleoside content.

In the minicircles of the invention, the at least one inverted repeat is essentially not truncated, compared to the wild-type inverted repeat of the virus of origin. As used herein, "essentially not truncated" is understood to mean that an inverted repeat is not truncated by more than 10% compared to the wild-type inverted repeat, such as by 10%, 9%, 8%, 7%, 6%, 5%, 4%, 3%, 2%, 1%, or less than 1%.

In one embodiment, the minicircle of the invention may further comprise a cargo nucleic acid. The inverted repeat may be located at any terminal end of the cargo sequence. In a preferred embodiment, the at least one inverted repeat is at least one ITR.

In a preferred embodiment, an inverted repeat is at least one ITR at the 5' terminus of a cargo nucleic acid. In another preferred embodiment, an inverted repeat is at least one ITR at the 3' terminus of a cargo nucleic acid.

In a preferred embodiment, the plasmid contains two ITRs located at the two termini of the cargo nucleic acid. In a preferred embodiment, the cargo nucleic acid contains at least one ITR located at the 5' terminus of the coding strand of the cargo nucleic acid and at least one ITR located at the 3' terminus of the coding strand of the cargo nucleic acid.

In a preferred embodiment, the cargo nucleic acid comprises or consists of a sequence encoding for:
viral polypeptides and/or nucleic acids, preferably one or more adeno-associated virus (AAV) polypeptides and/or nucleic acids, preferably a whole adeno-associated virus (AAV), in particular an AAV serotype 2;
RNA, in particular messenger RNA, small interfering RNA and/or trans-activating crRNA; and/or
DNA or interest, in particular DNA encoding for a polypeptide of interest.

In another aspect, the invention relates to a composition comprising a population of minicircles as described above, wherein the inverted repeats of the population of minicircles have an average sequence identity of at least 90%, at least 92%, or at least 95% to the corresponding wild-type inverted repeat of the virus of origin.

In a preferred embodiment, at least 50%, at least 60%, at least 70%, at least 80%, at least 90%, at least 95%, at least 98%, at least 99%, or 100% of inverted repeats in the population of minicircles are not truncated.

### Methods for producing minicircles

Minicircles can be produced by recombination from corresponding plasmids, as described in WO 96/26270 or EP 1620559. Generally, production starts with a plasmid ("parental plasmid") that contains the complete sequence of the desired minicircle (minicircle region) and additionally a plasmid backbone (miniplasmid region). Recombinase recognition sequences are located on both sides between the minicircle region and the miniplasmid region, which are specifically recognized by a corresponding recombinase. A recombinase specific for given recombinase recognition sequences is any enzyme that can catalyze direction-specific recombination at the recombinase recognition sequences.

The parental plasmid is recombined by the specific recombinase into a minicircle and a remaining residue, the miniplasmid. The two recombinase recognition sequences must be oriented in such a way that the recombination results in separation into a minicircle and a miniplasmid, rather than creating a single modified plasmid.

Examples of possible recombinases are the integrases from bacteriophages λ (see Landy *et al.*; 7), P22 and Φ80 (see Leong *et al.*; 8), the HP1 integrase from *Haemophilus influenzae* (see Hauser *et al.*; 6), the Cre integrase from phage P1, the integrase from plasmid pSAM2 (see EP350341), the Flp recombinase from plasmid 2µ (see Andrews *et al.*; 1), or the ΦC31 integrase (see Bateman *et al.*; 2). Alternatively, recombinases from transposons of the Tn3 family can be used, such as the parA resolvase from RP4 (see Eberl *et al.*; 5), the resolvases of transposons Tn3, Tn21, or Tn522 (see Stark *et al.*; 18), or the Gin invertase from bacteriophage µ.

Recombination of the parental plasmid can occur *in vitro* or *in vivo*. One advantage of *in vivo* recombination in host cells is that the parental plasmid does not need to be purified, provided expression of the corresponding recombinase can be induced in the host cells. Preferably, expression of the recombinase is inducible, for example, via a change in temperature or the addition of a metabolite. After induction of recombinase expression in the host cell, the parental plasmid is cleaved into two supercoiled, circular molecules, the minicircle and the miniplasmid, which can be separated from each other after cell lysis. Methods for the production of minicircles are described for instance in DE 102011118018 B4.

Accordingly, another aspect of the invention relates to a method for producing a minicircle from a parental plasmid, wherein the minicircle comprises at least one inverted repeat, the method comprising the steps:
(i) transforming *Escherichia coli* (*E. coli*) cells with said parental plasmid;
(ii) continuously cultivating said *E. coli* cells at a temperature of at least 39°C, comprising propagating the parental plasmid of step (i); and
(iii) inducing the formation of minicircles from the propagated parental plasmid of step (ii).

The minicircle produced may be a minicircle of the invention as described above, comprising an inverted repeat of viral origin. However, the inverted repeat may also be of any other origin, such as of bacterial origin, of human or animal origin, or plant origin, of fungal origin, of synthetic origin, possibly aided by computational design, or of a combination of two or more thereof.

In a preferred embodiment, an inverted repeat is of viral origin, such as of AAV origin, in particular of AAV serotype 2. In a particularly preferred embodiment, the at least one inverted repeat is an ITR.

In a preferred embodiment, the inverted repeat is characterized in that it is at least 20 bp in length, at least 10 bp in length, at least 25 bp in length, at least 50 bp in length, at least 75 bp in length, at least 80 bp in length, at least 90 bp in length, at least 93 bp in length, at least 94 bp in length, at least 100 bp in length, at least 110 bp in length, at least 120 bp in length, at least 130 bp in length, or at least 140 bp in length, or at least 150 bp in length.

In a preferred embodiment, the inverted repeat is characterized in that it is not more than 1000 bp in length, not more than 300 bp in length, not more than 200 bp in length, not more than 150 bp in length, not more than 100 bp in length, or not more than 50 bp in length.

The molar content of guanosine and cytidine (guanosine/cytidine content, GC content), based on the overall nucleoside content, of the inverted repeat may be any content. In a preferred embodiment, the inverted repeat is characterized in that it has a guanosine/cytidine content of at least 50 mol%, at least 60 mol%, at least 65 mol%, at least 70 mol%, at least 75 mol%, or at least 80 mol%, based on the overall nucleoside content.

In a preferred embodiment, the inverted repeat is characterized in that:
it is of viral origin, in particular AAV origin;
it is an ITR, in particular an ITR of AAV origin;
it is at least 131 base pairs in length including the D-sequence; and/or
it has a guanosine/cytidine content of at least 70 mol%, based on the overall nucleoside content.

In a preferred embodiment, said inverted repeat is essentially not truncated, preferably wherein said inverted repeat has a sequence identity of at least 90%, at least 92%, or at least 95% compared to the corresponding inverted repeat on the parental plasmid.

In another preferred embodiment, the inverted repeats of the of the minicircles obtained in step (iii) have an average sequence identity of at least 90%, at least 92%, or at least 95% to the corresponding inverted repeat in the minicircle region of the parental plasmid.

In one embodiment, the minicircles obtained in step (iii) may further comprise a cargo nucleic acid. The inverted repeat may be located at any terminal end of the cargo sequence. In a preferred embodiment, the at least one inverted repeat is at least one ITR.

In a preferred embodiment, an inverted repeat is at least one ITR at the 5' terminus of a cargo nucleic acid. In another preferred embodiment, an inverted repeat is at least one ITR at the 3' terminus of a cargo nucleic acid.

In a preferred embodiment, the minicircle contains two ITRs located at the two termini of the cargo nucleic acid. In a preferred embodiment, the cargo nucleic acid contains at least one ITR located at the 5' terminus of the coding strand of the cargo nucleic acid and at least one ITR located at the 3' terminus of the coding strand of the cargo nucleic acid.

In a preferred embodiment, the cargo nucleic acid comprises or consists of a sequence encoding for:
viral polypeptides and/or nucleic acids, preferably one or more adeno-associated virus (AAV) polypeptides and/or nucleic acids, preferably a whole adeno-associated virus (AAV), in particular an AAV serotype 2;
RNA, in particular messenger RNA, small interfering RNA and/or trans-activating crRNA; and/or DNA or interest, in particular DNA encoding for a polypeptide of interest.

In a preferred embodiment, the parental plasmid contains at least one sequence mediating antibiotic resistance. In a preferred embodiment, such sequence mediating antibiotic resistance may be a sequence encoding for an enzyme that facilitates at least partial inactivation of an antibiotic. For instance, kanamycin (Kan) and nptII or beta-lactamase facilitating the cleavage of a beta-lactam cycle of beta-lactam antibiotics. This enables selecting Escherichia coli comprising the at least one plasmid, because only those bacteria containing the plasmid will be widely resistant against the antibiotic.

In another embodiment, the parental plasmid contains at least one sequence encoding for an enzyme that enables growth in the absence of an essential nutrient component. For instance, a deficiency mutant of the bacteria which is not autotrophic for a certain nutrient such as for an amino acid may only grow in a medium that is essentially lacking this nutrient, when the plasmid that cures the deficiency is present in the bacterium. This enables selecting Escherichia coli comprising the at least one plasmid.

The *E. coli* cells may be any *Escherichia coli* bacteria known in the art. In a preferred embodiment, the *E. coli* bacteria may be a strain that may be cultivated at a temperature of up to at least 42°C, up to at least 43°C, up to at least 44°C, up to at least 45°C, up to at least 46°C, or even above 46°C. In a preferred embodiment, the *E. coli* bacteria may be a strain that may be able to proliferate at least at a temperature of 10 to 46°C, of 15 to 45°C, of 18 to 44°C, of 20 to 43.5°C, of 25 to 43°C, of 30 to 42°C, or of 35 to 42°C.

In a preferred embodiment, the *E. coli* bacteria may be any strain that is not heat-labile at the intended temperature. In a preferred embodiment, the *E. coli* bacteria are wild-type *E. coli* bacteria. In a preferred embodiment, the *E. coli* bacteria are K-12 derivative Escherichia coli bacteria. In a preferred embodiment, the *E. coli* bacteria are B derivative *E. coli* bacteria. In another preferred embodiment, the *E. coli* cells have the genotype sbcC⁻ or sbcD⁻. In another preferred embodiment, the *E. coli* cells comprise a chromosomal recA⁻ and/or endA⁻ mutation. In another preferred embodiment, the *E. coli* cells comprise a chromosomal recA⁻ and/or endA⁻ mutation in an sbcC⁻ or sbcD⁻ genotype.

Strains of *E. coli* bacteria are available that are adapted to cultivation at high temperatures up to 48.5°C as shown, for example, in Rudolph *et al.* (13). Such bacteria strains may, for instance, be used when higher temperature ranges are of interest.

In a preferred embodiment, the *E. coli* bacteria may be adapted to maintain proliferation at an elevated temperature above 37°C, above 38°C, above 39°C, above 40°C, above 41°C, above 42°C, above 43°C, or above 44°C by selecting bacteria using cultivating over several proliferation cycles at such elevated temperature.

In another embodiment, the *E. coli* bacteria are not adapted to growth at elevated temperatures and are subjected to elevated temperatures only after transformation.

Step (i) of transforming *E. coli* cells with the parental plasmid may be performed by any means. The skilled person is aware of a variety of means for introducing a plasmid into an *E. coli* cell, such as chemical transformation or electroporation.

Step (ii) of cultivating the *E. coli* cells of step (i) may be conducted by any means. The person skilled in the art will be aware of a large variety of cell culture methods. Cultivating may be conducted in a liquid culture, a culture using a gel-like cell culture plate (e.g., agar plate), or a sequential combination thereof.

In a preferred embodiment, step (ii) of cultivating the *E. coli* cells is conducted for at least 16 hours, for at least 24 hours, for at least 36 hours, for at least 48 hours, or for at least three days. When the step (ii) of cultivating the *E. coli* cells is conducted for more than 16 hours, the cultivating exceeding 16 hours may be optionally conducted at the same temperature or at a different temperature such as a lower temperature.

In a preferred embodiment, the *E. coli* bacteria comprising the parental plasmid are not incubated for more than 12 hours, preferably not incubated for more than 6 hours, in particular not incubated for more than 3 hours, at a temperature below 39°C before step (ii) is conducted. In a preferred embodiment, the *E. coli* bacteria comprising the parental plasmid are not incubated for more than 12 hours, preferably not incubated for more than 6 hours, in particular not incubated for more than 3 hours, at a temperature below 39°C after step (ii) is conducted. In a preferred embodiment, the cultivating step (ii) is (essentially) the only cultivating step conducted with the *E. coli* bacteria comprising the parental plasmid. It will be understood that the bacteria may optionally be cultivated at lower temperatures before the parental plasmid is introduced.

In a preferred embodiment, step (ii) of cultivating the *E. coli* cells is conducted for at least 16 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells is conducted for at least 24 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells is conducted for at least 36 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells is conducted for at least 48 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells is conducted for at least three days at a temperature of at least 39.5°C, more preferably of at least 40°C, of at least 40.5°C, of at least 41°C, of at least 41.5°C, or of at least 42°C.

In a preferred embodiment, step (ii) of cultivating the *E. coli* cells is conducted for at least 16 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells is conducted for at least 24 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells is conducted for at least 36 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells is conducted for at least 48 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells is conducted for at least three days at a temperature in the range of from 39°C to 46°C or of from 40°C to 45°C or of from 41°C to 44°C or of from 41°C to 43°C or at 42°C.

In a preferred embodiment, the *E. coli* cells are permanently cultivated at the temperature ranges as indicated above. In an alternative preferred embodiment, the *E. coli* cells are cultivated at different temperatures including at least one time interval of cultivating at the temperature ranges at indicated above.

Step (iii) of inducing formation of minicircles from the propagated parental plasmids may be performed using any means known in the art. As described above, expression of a recombinase may be induced in the *E. coli* cells, for example via a change in temperature or the addition of a metabolite. After induction of recombinase expression in the *E. coli* cells, the parental plasmid is cleaved into two supercoiled, circular molecules, the minicircle and the miniplasmid. The required gene encoding the recombinase can be present on a different nucleic acid molecule in the *E. coli* cells. It is particularly advantageous if the recombinase gene is present on the parental plasmid within the miniplasmid region.

The method may optionally contain one or more further steps conducted subsequent to step (iii) of inducing the formation of minicircles.

In a preferred embodiment, the method further comprises a step (iv) of isolating the minicircles. The isolating of the minicircles may be conducted by any means known in the art. Standard nucleic acid isolation techniques may be used to isolate plasmid DNA comprising minicircles and miniplasmids from the *E. coli* cells. Minicircles and miniplasmids may be separated, for instance, by affinity chromatography, as described by Mayrhofer *et al.* (10).

### Methods for propagating plasmids

The present disclosure also provides methods for the propagation of plasmids containing inverted repeats on a large scale, using an elevated cultivation temperature in order to maintain the integrity of the inverted repeats.

Accordingly, in a further aspect, the invention relates to a method of propagating a plasmid in *E. coli* cells, wherein the plasmid comprises at least one inverted repeat, the method comprising the steps:
(i) transforming *E. coli* cells with said plasmid;
(ii) continuously cultivating the *E. coli* cells comprising the plasmid of step (i) in a pre-culture at a temperature of at least 39°C for at least 8 hours, preferably for at least 16 hours; and
(iii) inoculating a main culture with *E.* coli cells from the pre-culture of step (ii) and cultivating the main culture at a temperature of at least 39°C.

In a preferred embodiment, the main culture is a large scale culture in a fermenter. The main culture may have a volume of at least 5 l, at least 10 l, at least 20 l, at least 30 l, at least 50 l, at least 75 l, at least 100 l, at least 200 l, at least 300 l, at least 500 l, or at least 1000 l.

The plasmid may be a parental plasmid suitable for the production of minicircles, as described above. Alternatively, the plasmid may be any other plasmid comprising an inverted repeat. The plasmid may be circular or linear. In a preferred embodiment, the plasmid is a circular plasmid. In a preferred embodiment, the plasmid is a circular plasmid of double-stranded DNA. In a preferred embodiment, the plasmid is a circular plasmid of double-stranded DNA of at least 1000 base pairs in length, preferably of at least 2000 base pairs in length, in particular of at least 3000 base pairs in length.

In a preferred embodiment, the plasmid is a circular plasmid of double-stranded DNA of 100 to 100000 base pairs in length, of 500 to 50000 base pairs in length, of 750 to 25000 base pairs in length, of 1000 to 10000 base pairs in length, of 1500 to 7500 base pairs in length, or of 2000 to 5000 base pairs in length.

The inverted repeat may also be of any origin, such as of viral origin, bacterial origin, of human or animal origin, or plant origin, of fungal origin, of synthetic origin, possibly aided by computational design, or of a combination of two or more thereof.

In a preferred embodiment, an inverted repeat is of viral origin, such as of AAV origin, in particular of AAV serotype 2. In a particularly preferred embodiment, the at least one inverted repeat is an ITR.

In a preferred embodiment, the inverted repeat is characterized in that it is at least 20 bp in length, at least 10 bp in length, at least 25 bp in length, at least 50 bp in length, at least 75 bp in length, at least 80 bp in length, at least 90 bp in length, at least 93 bp in length, at least 94 bp in length, at least 100 bp in length, at least 110 bp in length, at least 120 bp in length, at least 130 bp in length, or at least 140 bp in length, or at least 150 bp in length.

In a preferred embodiment, the inverted repeat is characterized in that it is not more than 1000 bp in length, not more than 300 bp in length, not more than 200 bp in length, not more than 150 bp in length, not more than 100 bp in length, or not more than 50 bp in length.

The molar content of guanosine and cytidine (guanosine/cytidine content, GC content), based on the overall nucleoside content, of the inverted repeat may be any content. In a preferred embodiment, the inverted repeat is characterized in that it has a guanosine/cytidine content of at least 50 mol%, at least 60 mol%, at least 65 mol%, at least 70 mol%, at least 75 mol%, or at least 80 mol%, based on the overall nucleoside content.

In a preferred embodiment, the inverted repeat is characterized in that:
it is of viral origin, in particular AAV origin;
it is an ITR, in particular an ITR of AAV origin;
it is at least 131 base pairs in length including the D-sequence; and/or
it has a guanosine/cytidine content of at least 70 mol%, based on the overall nucleoside content.

In a preferred embodiment, said inverted repeat is essentially not truncated, preferably wherein said inverted repeat has a sequence identity of at least 90%, at least 92%, or at least 95% compared to the corresponding inverted repeat on the parental plasmid.

In a preferred embodiment, at least 50%, at least 60%, at least 70%, at least 80%, at least 90%, at least 95%, at least 98%, at least 99%, or 100% of inverted repeats are essentially not truncated after 1 day of cultivating in accordance with steps (ii) and (iii), compared to the inverted repeat of the plasmid of step (i).

In a preferred embodiment, at least 50%, at least 60%, at least 70%, at least 80%, at least 90%, at least 95%, at least 98%, at least 99%, or 100% of inverted repeats are essentially not truncated after 3 days of cultivating in accordance with steps (ii) and (iii), compared to the inverted repeat of the plasmid of step (i).

In a preferred embodiment, at least 80% of inverted repeats are essentially not truncated after 3 days of cultivating in accordance with steps (ii) and (iii), compared to the inverted repeat of the plasmid of step (i) and/or at least 80% of inverted repeats are essentially not truncated after 3 days of cultivating in accordance with steps (ii) and (iii), compared to the inverted repeat of the plasmid of step (i).

In a preferred embodiment, after 1 day of cultivation in step (ii) and step (iii), the inverted repeats of the plasmids in the main culture of step (iii) have an average sequence identity of at least 90%, at least 92%, or at least 95% compared to the inverted repeat of the plasmid of step (i).

In a preferred embodiment, after 3 days of cultivation in step (ii) and step (iii), the inverted repeats of the plasmids in the main culture of step (iii) have an average sequence identity of at least 90%, at least 92%, or at least 95% compared to the inverted repeat of the plasmid of step (i).

In one embodiment, the plasmid may further comprise a cargo nucleic acid. The inverted repeat may be located at any terminal end of the cargo sequence. In a preferred embodiment, the at least one inverted repeat is at least one ITR.

In a preferred embodiment, an inverted repeat is at least one ITR at the 5' terminus of a cargo nucleic acid. In another preferred embodiment, an inverted repeat is at least one ITR at the 3' terminus of a cargo nucleic acid.

In a preferred embodiment, the plasmid contains two ITRs located at the two termini of the cargo nucleic acid. In a preferred embodiment, the cargo nucleic acid contains at least one ITR located at the 5' terminus of the coding strand of the cargo nucleic acid and at least one ITR located at the 3' terminus of the coding strand of the cargo nucleic acid.

In a preferred embodiment, the cargo nucleic acid comprises or consists of a sequence encoding for:
viral polypeptides and/or nucleic acids, preferably one or more adeno-associated virus (AAV) polypeptides and/or nucleic acids, preferably a whole adeno-associated virus (AAV), in particular an AAV serotype 2;
RNA, in particular messenger RNA, small interfering RNA and/or trans-activating crRNA; and/or DNA or interest, in particular DNA encoding for a polypeptide of interest.

In a preferred embodiment, the plasmid contains at least one sequence mediating antibiotic resistance. In a preferred embodiment, such sequence mediating antibiotic resistance may be a sequence encoding for an enzyme that facilitates at least partial inactivation of an antibiotic. For instance, kanamycin (Kan) and nptII or beta-lactamase facilitating the cleavage of a beta-lactam cycle of beta-lactam antibiotics. This enables selecting Escherichia coli comprising the at least one plasmid, because only those bacteria containing the plasmid will be widely resistant against the antibiotic.

In another embodiment, the plasmid contains at least one sequence encoding for an enzyme that enables growth in the absence of an essential nutrient component. For instance, a deficiency mutant of the bacteria which is not autotrophic for a certain nutrient such as for an amino acid may only grow in a medium that is essentially lacking this nutrient, when the plasmid that cures the deficiency is present in the 10 bacterium. This enables selecting Escherichia coli comprising the at least one plasmid.

The *E. coli* cells may be any *Escherichia coli* bacteria known in the art. In a preferred embodiment, the *E. coli* bacteria may be a strain that may be cultivated at a temperature of up to at least 42°C, up to at least 43°C, up to at least 44°C, up to at least 45°C, up to at least 46°C, or even above 46°C. In a preferred embodiment, the *E. coli* bacteria may be a strain that may be able to proliferate at least at a temperature of 10 to 46°C, of 15 to 45°C, of 18 to 44°C, of 20 to 43.5°C, of 25 to 43°C, of 30 to 42°C, or of 35 to 42°C.

In a preferred embodiment, the *E. coli* bacteria may be any strain that is not heat-labile at the intended temperature. In a preferred embodiment, the *E. coli* bacteria are wild-type *E. coli* bacteria. In a preferred embodiment, the *E. coli* bacteria are K-12 derivative Escherichia coli bacteria. In a preferred embodiment, the *E. coli* bacteria are B derivative *E. coli* bacteria. In another preferred embodiment, the *E. coli* cells have the genotype sbcC⁻ or sbcD⁻. In another preferred embodiment, the *E. coli* cells comprise a chromosomal recA⁻ and/or endA⁻ mutation.

In another preferred embodiment, the *E. coli* cells comprise a chromosomal recA⁻ and/or endA⁻ mutation in an sbcC⁻ or sbcD⁻ genotype.

Strains of *E. coli* bacteria are available that are adapted to cultivation at high temperatures up to 48.5°C as shown, for example, in Rudolph *et al.* (13). Such bacteria strains may, for instance, be used when higher temperature ranges are of interest.

In a preferred embodiment, the *E. coli* bacteria may be adapted to maintain proliferation at an elevated temperature above 37°C, above 38°C, above 39°C, above 40°C, above 41°C, above 42°C, above 43°C, or above 44°C by selecting bacteria using cultivating over several proliferation cycles at such elevated temperature.

In another embodiment, the *E. coli* bacteria are not adapted to growth at elevated temperatures and are subjected to elevated temperatures only after transformation.

Step (i) of transforming *E. coli* cells with the plasmid may be performed by any means. The skilled person is aware of a variety of means for introducing a plasmid into an *E. coli* cell, such as heat shock transformation or electroporation.

Step (ii) of cultivating the *E. coli* cells of step (i) in a pre-culture may be conducted by any means. The person skilled in the art will be aware of a large variety of cell culture methods. Cultivating may be conducted in a liquid culture, a culture using a gel-like cell culture plate (e.g., agar plate), or a sequential combination thereof.

In a preferred embodiment, step (ii) of cultivating the *E. coli* cells in a pre-culture is conducted for at least 16 hours, for at least 24 hours, for at least 36 hours, for at least 48 hours, or for at least three days. When the step (ii) of cultivating the *E. coli* cells is conducted for more than 16 hours, the cultivating exceeding 16 hours may be optionally conducted at the same temperature or at a different temperature such as a lower temperature.

In a preferred embodiment, the *E. coli* bacteria comprising the plasmid are not incubated for more than 12 hours, preferably not incubated for more than 6 hours, in particular not incubated for more than 3 hours, at a temperature below 39°C before step (ii) is conducted. In a preferred embodiment, the *E. coli* bacteria comprising the parental plasmid are not incubated for more than 12 hours, preferably not incubated for more than 6 hours, in particular not incubated for more than 3 hours, at a temperature below 39°C after step (ii) is conducted. It will be understood that the bacteria may optionally be cultivated at lower temperatures before the plasmid is introduced.

In a preferred embodiment, step (ii) of cultivating the *E. coli* cells in a pre-culture is conducted for at least 16 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells in a pre-culture is conducted for at least 24 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells in a pre-culture is conducted for at least 36 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells in a pre-culture is conducted for at least 48 hours at a temperature of at least 39.5°C, more preferably of at least 40°C, of at least 40.5°C, of at least 41°C, of at least 41.5°C, or of at least 42°C. In a preferred embodiment, step (ii) of cultivating the *E. coli* cells in a pre-culture is conducted for at least three days at a temperature of at least 39.5°C, more preferably of at least 40°C, of at least 40.5°C, of at least 41°C, of at least 41.5°C, or of at least 42°C.

In a preferred embodiment, step (ii) of cultivating the *E. coli* cells in a pre-culture is conducted for at least 16 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells in a pre-culture is conducted for at least 24 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells in a pre-culture is conducted for at least 36 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells in a pre-culture is conducted for at least 48 hours at a temperature in the range of from 39°C to 46°C or of from 40°C to 45°C or of from 41°C to 44°C or of from 41°C to 43°C or at 42°C. In a preferred embodiment, step (ii) of cultivating the *E. coli* cells in a pre-culture is conducted for at least three days at a temperature in the range of from 39°C to 46°C or of from 40°C to 45°C or of from 41°C to 44°C or of from 41°C to 43°C or at 42°C.

In a preferred embodiment, the *E. coli* cells are permanently cultivated at the temperature ranges as indicated above. In an alternative preferred embodiment, the *E. coli* cells are cultivated at different temperatures including at least one time interval of cultivating at the temperature ranges at indicated above.

Step (iii) of cultivating a main culture is performed in liquid culture. The liquid culture preferably has a volume of at least 5 l, at least 10 l, at least 20 l, at least 30 l, at least 50 l, at least 75 l, at least 100 l, at least 200 l, at least 300 l, at least 500 l, or at least 1000 l. Preferably, step (iii) is performed in an industrial fermenter.

Step (iii) of cultivating a main culture is conducted for at least 3 hours, for at least 6 hours, for at least 8 hours, for at least 10 hours, for at least 12 hours, for at least 16 hours, for at least 24 hours, for at least 36 hours, for at least 48 hours, or for at least three days.

In a preferred embodiment, step (iii) of cultivating a main culture is conducted for at least 6 hours at a temperature of at least 39.5°C, more preferably of at least 40°C, of at least 40.5°C, of at least 41°C, of at least 41.5°C, or of at least 42°C. In a preferred embodiment, step (iii) of cultivating a main culture is conducted for at least 8 hours at a temperature of at least 39.5°C, more preferably of at least 40°C, of at least 40.5°C, of at least 41°C, of at least 41.5°C, or of at least 42°C. In a preferred embodiment, step (iii) of cultivating a main culture is conducted for at least 16 hours at a temperature of at least 39.5°C, more preferably of at least 40°C, of at least 40.5°C, of at least 41°C, of at least 41.5°C, or of at least 42°C. In a preferred embodiment, step (iii) of cultivating a main culture is conducted for at least 24 hours at a temperature of at least 39.5°C, more preferably of at least 40°C, of at least 40.5°C, of at least 41°C, of at least 41.5°C, or of at least 42°C. In a preferred embodiment, step (iii) of cultivating a main culture is conducted for at least 36 hours at a temperature of at least 39.5°C, more preferably of at least 40°C, of at least 40.5°C, of at least 41°C, of at least 41.5°C, or of at least 42°C.

In a preferred embodiment, step (iii) of cultivating a main culture is conducted for at least 6 hours at a temperature in the range of from 39°C to 46°C or of from 40°C to 45°C or of from 41°C to 44°C or of from 41°C to 43°C or at 42°C. In a preferred embodiment, step (iii) of cultivating a main culture is conducted for at least 8 hours at a temperature in the range of from 39°C to 46°C or of from 40°C to 45°C or of from 41°C to 44°C or of from 41°C to 43°C or at 42°C. In a preferred embodiment, step (iii) of cultivating a main culture is conducted for at least 16 hours at a temperature in the range of from 39°C to 46°C or of from 40°C to 45°C or of from 41°C to 44°C or of from 41°C to 43°C or at 42°C. In a preferred embodiment, step (iii) of cultivating a main culture is conducted for at least 24 hours at a temperature in the range of from 39°C to 46°C or of from 40°C to 45°C or of from 41°C to 44°C or of from 41°C to 43°C or at 42°C. In a preferred embodiment, step (iii) of cultivating a main culture is conducted for at least 36 hours at a temperature in the range of from 39°C to 46°C or of from 40°C to 45°C or of from 41°C to 44°C or of from 41°C to 43°C or at 42°C.

In a preferred embodiment, the *E. coli* cells are permanently cultivated at the temperature ranges as indicated above. In an alternative preferred embodiment, the *E. coli* cells are cultivated at different temperatures including at least one time interval of cultivating at the temperature ranges at indicated above.

When the plasmid is a parental plasmid for the production of minicircles, the method may further comprise a step (iv) of inducing the formation of minicircles from said parental plasmid in the main culture of step (iii), wherein step (iv) is performed for at least 30 minutes, at least 1 hour, preferably for 1-4 hours or 4-8 hours.

Step (iv) of inducing formation of minicircles from the propagated parental plasmids may be performed using any means known in the art. As described above, expression of a recombinase may be induced in the *E. coli* cells, for example via a change in temperature or the addition of a metabolite. After induction of recombinase expression in the *E. coli* cells, the parental plasmid is cleaved into two supercoiled, circular molecules, the minicircle and the miniplasmid. The required gene encoding the recombinase can be present on a different nucleic acid molecule in the *E. coli* cells. It is particularly advantageous if the recombinase gene is present on the parental plasmid within the miniplasmid region.

The method may optionally contain one or more further steps conducted subsequent to step (iv) of inducing the formation of minicircles.

In a preferred embodiment, the method further comprises a step (v) of isolating the minicircles. The isolating of the minicircles may be conducted by any means known in the art. Standard nucleic acid isolation techniques may be used to isolate plasmid DNA comprising minicircles and miniplasmids from the *E. coli* cells. Minicircles and miniplasmids may be separated, for instance, by affinity chromatography, as described by Mayrhofer *et al.* (10).

### EXAMPLES

### EXAMPLE 1

### Prevalence of truncated ITR sequences in the art

The state of the art regarding inverted repeat stability in recombinant *E. coli* plasmids is exemplified through a meta-analysis on the integrity of ITRs in plasmids available in the art. Sequences of 188 ITR-encoding plasmids were programmatically downloaded from the Addgene database (addgene.org, accessed 17 January 2023, all plasmids satisfying the search terms "AAV" and "ITR" available from Addgene at the time of analysis). 123 of these plasmids had been sequence-verified by Addgene and were further analyzed (Addgene IDs 104839, 105669, 105677, 105678, 105679, 113763, 120219, 121502, 121921, 125713, 126520, 126521, 129267, 135964, 135965, 135966, 135967, 137188, 139979, 139980, 30 154849, 154871, 160069, 163791, 163792, 163793, 163794, 163795, 163796, 163797, 164468, 165441, 166870, 166871, 166872, 166873, 167577, 167578, 167579, 167580, 167581, 170117, 170118, 170120, 170121, 170122, 170123, 174703, 180183, 180184, 180191, 180192, 180193, 183078, 183079, 183081, 184002, 184003, 186418, 186419, 187181, 187182, 190473, 190474, 190475, 35 191523, 191795, 192569, 192570, 192572, 192573, 192575, 192576, 193166, 193168, 193169, 193170, 193171, 193172, 193174, 193175, 193176, 193177, 193178, 193179, 193180, 193181, 193182, 193183, 193185, 193186, 193187, 193188, 26968, 26969, 26971, 26972, 26973, 26975, 26976, 50455, 50464, 50466, 60226, 60227, 60229, 65417, 65418, 82705, 85451, 86949, 86950, 86954, 86955, 87951, 89256, 89570, 98216, 98217, 98218, 98219, 98220, 98704). Mapping analysis revealed that, of these plasmids, 122 had ITRs with wild-type D sequences, but none had full-length ITRs, and no ITR exceeded 130 bp (wild-type AAV ITRs are more than 140 bp in length). Furthermore, of the sequence-verified plasmids, 75 ITRs (encoded on 69 plasmids) had an internal deletion, and the internal deletions were 11 bp long in 73 cases and 22 bp and 25 bp long in one case each. Six plasmids had internal deletions in both ITRs. Consequently, the findings indicate that plasmids with stable, full-length ITRs are currently challenging to obtain.

### EXAMPLE 2

### Impact of E. coli propagation at 37°C on ITR integrity

A sequencing approach (R9 pore chemistry) was used to investigate the impact of cloning and propagation in *E. coli* (DH5α, 37°C, LB Lennox) on the integrity of the ITRs. A plasmid, pZMB938, was designed containing two full-length ITRs from AAV2. The origin proximal ITR 145 bp identical to the AAV2 reference genome (NC_001401), while the origin distal ITR comprised 143 bp, with two A nucleotides missing toward the terminus. Nevertheless, both ITRs were treated as full-length ITRs for the purposes of this study.

After the first overnight culture starting from a single colony (termed day 1), the origin distal ITR in the originally obtained plasmid stock (consensus 143 bp) was called at 134 bp ± 9 bp, and the origin proximal ITR was called at 120 bp ± 26 bp (n = 208), indicating that, at least, the origin proximal ITR was already severely truncated in the cloning and propagation procedure. The culture was propagated for a total of seven days, diluting 10 µl overnight culture to 5 ml each morning, and the analysis was repeated (n average 30 = 124). The origin distal ITR showed moderately significant signs of degradation. However, the distributions for day one and day seven were not significant (origin distal ITR, t-test for day one to day x: p1-2 = 0.0009, p1-3 = 0.04, p1-4 = 0.001, p1-5 = 0.004, p1-6 = 0.003, p1-7 = 0.21). Using the normalized histogram subtraction method (normalization to total read count, 5 nt bin width), the degradation extent compared to the origin distal ITR on day 1 can be estimated for significantly different distributions. For the origin distal ITR, at most, 20% degradation was observed (day 2). Distributions for the origin proximal ITR were all significantly different compared to the origin distal ITR on day 1, and the extent of degradation was estimated to be at most 49% (days 1 - 7: 32%, 37%, 34%, 44%, 41%, 48%, 42%). These findings indicate that wild-type ITR sequences are unstable in *E. coli* DH5α, cultivated under standard conditions. The ability to clone these sequences was probably due to screening many clones, which will be a mixture of variants. The mixture will also be present after each single overnight culture starting from a single colony.

The analysis was repeated with *E. coli* Stbl3 (Thermo Fisher #C737303, 37°C, LB Lennox), starting from a single colony after transformation with the originally obtained plasmid stock. As before, several clones were initially investigated to find one with presumably intact ITRs since ITRs were already mostly degraded in the original stock, as described above. In the best-observed clone (day 1), the origin distal ITR was called at 133 bp ± 8 bp. The origin proximal ITR was called at 131 bp ± 12 bp (n = 353, p = 0.001, 12% degradation distal-to-proximal), indicating that the ITRs are more stable in *E. coli* Stbl3 over one day of culture, compared to *E. coli* DH5α. However, while the origin distal ITR, again, remained relatively stable in Stbl3 over the culture time (p1-2 = 0.003, p1-3 = 0.14, p1 4 = 0.02, p1-5 = 0.003, p1-6 = 0.0006, p1-7 = 0.03, at most 18% degradation on day 2 of the culture), the origin proximal ITR was more heavily damaged in Stbl3 over seven consecutive days, as compared to DH5α (degradation according to histogram subtraction: day 1: 12%, day 2: 39%, day 3: 47%, day 4: 54%, day 5: 60%, day 6: 61%, day 7: 65%). On day 7 of culture, the origin proximal ITR was called, on average, at 113 bp ± 22 bp. It can, therefore, not be concluded that an *E. coli* strain generally regarded as suitable for difficult sequences is adequate for maintaining these large inverted repeats and that inverted repeat stability is generally challenging in *E. coli* propagation.

### EXAMPLE 3

### Temperature-dependent stability of synthetic ITRs

Another plasmid, pZMB990, was generated, containing two synthetic ITRs of 144 bp flanking a GFP coding sequence.

A colony of *E. coli* Stbl3 transformed with pZMB990 and plated at 42°C was picked and propagated in LB medium 37°C or 42°C, respectively, for three days, and the ITR degradation status was determined as before, this time using the Oxford Nanopore Technology R10 sequencing chemistry. When propagated at 37°C for one day (plated at 42°C), the origin distal ITR was called at 142 bp ± 6 bp on 5 average, while the origin proximal ITR was called at 138 bp ± 14 bp, corresponding to 10% degradation. After three days of propagation at 37°C, there was no significant effect on the origin distal ITR (p = 0.21), but 32% of the origin proximal ITRs were degraded. When the culture was propagated at 42°C for one day, the origin distal ITR was called at 142 bp ± 6 bp, and the origin proximal ITR was called at 140 bp ± 12 bp (significant, 4% degradation). After three days of propagation at 42°C, the origin distal ITR was not significantly affected (p = 0.77), while the effect on the origin proximal ITR remained minor (significant, 5% degradation).

The effect of cultivation at 42°C, as opposed to standard cultivation at 37°C, on ITR stability is thus demonstrated to be 27% less ITR degradation in the case of plasmid pZMB990. Cultivation at elevated temperatures thus stabilizes full-length ITRs in *E. coli*.

### EXAMPLE 4

### Upscaling of the method to an industrial process

The industrially relevant *E. coli* production strain PF03 (having a mutation in the *sbcC* gene) was transformed with the plasmid pZMB990. 20 ng plasmid DNA were added to 100 µl of competent cells and incubated on ice for 20 min, followed by a heat shock at 42°C over 45 s in a mixing block. Then 900 µl SOC medium, pre-warmed to 42°C, was added to the cells. It is important to continuously maintain the elevated temperature of 42°C from this point onward. After quick mixing, the cells were plated onto ampicillin-supplemented agar plates, pre-warmed to 42°C, and incubated for 19 hours. Subsequently, several colonies were picked and shake flasks comprising 35 ml culture medium and 200 µg/ml ampicillin were inoculated. The flasks were incubated at 42°C.

After cultivation for 9.5 h, samples were taken from two of the colonies growing in the flasks for the isolation of plasmid DNA as an in-process control. 4 ml of the culture were centrifuged to separate the cells, and a commercial kit (Nucleospin Quickpure, Macherey Nagel) was used for isolation of the plasmids. The DNA was cut with restriction endonucleases SpeI and HindIII, and the band pattern was checked using agarose gel electrophoresis. After ensuring that the plasmid contained the origin proximal ITR sequence, the restriction reaction was checked using capillary gel electrophoresis. A single peak corresponding to the ITR-containing fragment proved that the plasmid population in culture was homogenous. The culture remaining in the shake flask was thus considered as a suitable for inoculation.

A fermenter with 30 l working volume of culture medium was prepared. An aeration rate of 1 vvm, an initial stirrer speed of 200 rpm, a pH of 7.0, and a temperature of 42°C were selected. The pH was automatically corrected by the addition of orthophosphoric acid or sodium hydroxide solution. The dissolved oxygen concentration was cascaded with the stirrer speed. After a total incubation for 14 h, the remaining 31 ml of culture in the shake flask were used to inoculate the fermenter by sterile injection. Batch fermentation was carried out in the absence of antibiotics. After 8 h batch fermentation, a sample was taken to measure the optical density of the culture and determine the volumetric concentration of the wet biomass. The bacterial culture was harvested after 9 h fermenter cultivation, while maintaining the bioreactor temperature at 42°C and slowly mixing at 200 rpm. First, 3 l of the bacterial culture were harvested into a pre-chilled 5 l beaker and immediately distributed among six pre-chilled centrifuge bottles. The culture was centrifuged at 4°C and 7000 rpm for 6 minutes. The reduced temperature during harvesting is intended to minimize the likelihood of plasmid replication by reducing the cells' metabolic activity as quickly as possible. Therefore, the DNA present in the biomass is expected to represent the state of the culture with respect to the integrity of the ITR sequence.

The first centrifugation step yielded 22.7 g of biomass, and a repeat centrifugation yielded 23.0 g of biomass. The remainder of the bacterial culture was harvested similarly and collected as a single mass of 203.6 g. The collected bacterial cells were packaged and immediately stored at -20 °C. Samples P1, P2, and P3 were taken from each of the collected biomass fractions. The DNA content of the cells was isolated using a commercial kit (Nucleosnap, Macherey Nagel), and the DNA was analyzed using the described nanopore sequencing method.

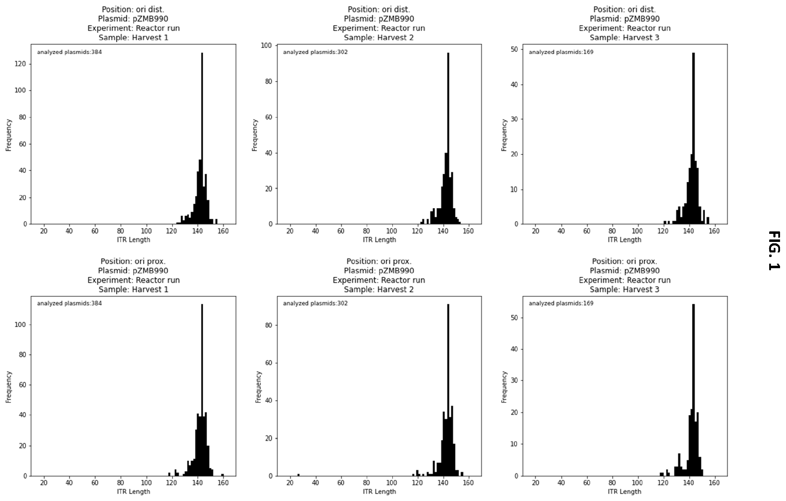

Sequencing showed that the ITRs were stably maintained during scaling to industrial process size and fermentation, as indicated by a narrow ITR length distribution around the expected 144 bp (Fig. 1). Importantly, it was possible to harvest the biomass at the end of a batch fermentation without compromising the quality of the DNA with regard to ITR sequence integrity. This aspect has implications for the technical implementation in larger-scale fermenters and for further adaptations. Thus, it was demonstrated that the method of the invention is transferable to an industrial process and large-scale production.

### EXAMPLE 5

### Production of minicircle DNA comprising full-length ITRs (ITR^{wt})

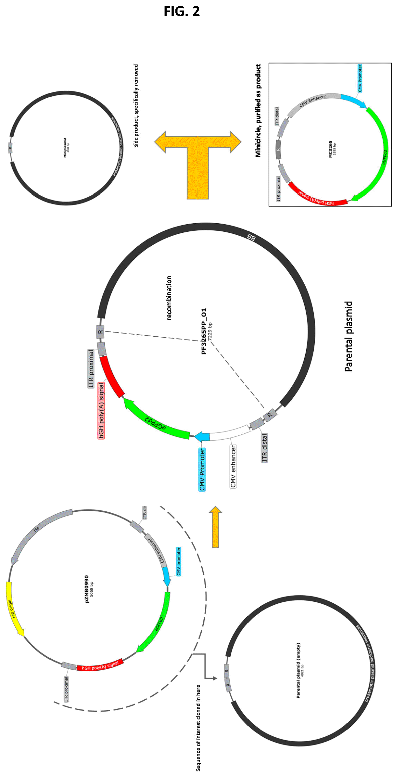

A minicircle is a derivative molecule in which the prokaryotic plasmid backbone sequences have been selectively removed. Using the technology applied by PlasmidFactory GmbH (Fig. 2), this results in a supercoiled monomeric construct consisting almost exclusively of the relevant sequence (sequence of interest) to be introduced into eukaryotic target cells or tissues. These contain the gene of interest and associated elements relevant for its expression, such as the promoter and polyA signal.

Other studies have shown that the production of recombinant AAV vectors using plasmid DNA as transfer, packaging, and helper constructs results in a significant amount of mispackaging of plasmid backbone sequences in the vectors. Replacing the plasmid DNA used for transfecting producer cells with minicircle DNA resulted in a significant improvement in the quality of AAV vectors. To combine this advantage with the aforementioned observation that full-length ITR sequences in the transfer construct lead to increased vector yield and quality, an approach was chosen to generate, for the first time, a minicircle with full-length ITR sequences that can be used as a transfer construct for AAV production. Thus the objective is to take advantage of the effects of the full-length ITR in the transfer construct (as described in DE 102023134327), resulting in far less mispackaging of bacterial backbone sequences, and combine them with the provision of a minicircle transfer construct wherein said bacterial backbone sequences have been removed previously.

First, the ITR-flanked region, along with the full-length ITRs, was cloned from the initial plasmid pZMB0990 into a parental plasmid (Fig. 2). 7.85 ng of parental plasmid PF3265PP were added to 50 µl of competent *E. coli* cells. The cells were shaken in SOC medium at 42°C for 15 min and then plated on LB-kanamycin plates pre-warmed to 42°C. After an overnight incubation, colonies were observed on the plates. For this construct, correct clones were then identified by colony PCR. For this purpose, 20 clones were picked and placed in culture tubes in liquid culture medium (4 ml) pre-warmed to 42°C. Cells were also taken from the same colonies and added to a colony PCR master mix (NEB). Primers were selected that bind to the miniplasmid region as well as to the cloned GOI. After 14 h, 400 µl of culture were taken from the tube from the best-growing clone identified as correct, and inoculated into six shake flasks, each containing 200 ml of culture medium and kanamycin at 75 µg/ml, as a pre-culture and shaken at 120 rpm. After 10.5 h cultivation, the pre-culture showed an optical density of more than 2 and was thus ready to be used as an inoculum for the main culture.

In-process control of the pre-culture was performed in parallel using capillary gel electrophoresis, and showed a single peak for the ITR-containing fragment. This served as the basis for confirming the selected clone and its plasmid composition regarding ITR homogeneity, before starting the minicircle production process.

The main culture was performed in three 5 l flasks, each containing a volume of 2.5 l culture medium. Each flask was inoculated with 400 ml pre-culture. Cultivation was performed at 42°C and shaking (150 rpm). After 5 h cultivation, the main culture had an optical density of 4.0. At this time, a pre-induction control was obtained from the main culture.

Then production of the minicircle was induced. After further cultivation for 30 min, cells were harvested by centrifugation. A post-induction sample was obtained from the centrifuged cells. In total, 54 g biomass were harvested from the three flasks.

The by-product, known as the miniplasmid, which consists of the plasmid backbone sequences, was selectively removed. The result was MC3265, a minicircle encoding a GFP reporter gene and containing full-length ITR sequences on both sides, which can be used as an AAV transfer minicircle. In total, 10.55 mg pure minicircle were obtained from the 54 g of biomass.

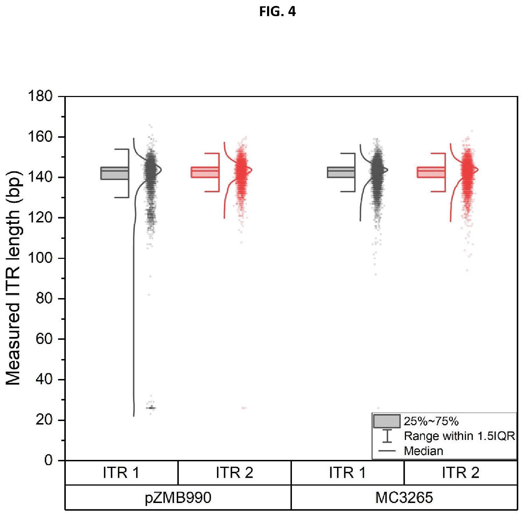

Minicircle MC3265 met all routine quality control specifications, including identity, concentration, supercoiled conformation, etc. (Fig.3). The key experimental result for the integrity of the ITR sequence came from Nanopore sequencing of the final minicircle product. We succeeded in combining the requirements for producing minicircles with those for maintaining the ITR sequences. It was found that the peak in the ITR length distribution was above 140 bp, with no evidence of a parallel peak in the distribution that would correspond to a truncated ITR (Fig. 4). Indeed, pZMB0990, which served as the starting molecule for this cloning, showed initial signs of degradation in the origin proximal ITR1, evident as a small hump at 120 bp in the ITR length distribution. The median ITR length for this plasmid was still above 140 bp. However, due to the selective effect of the cloning step and the subsequent maintenance of the culture 42°C, we observed that the minicircle generated from this plasmid showed a very sharp and distinct peak of the ITR length distribution at the full length of 144 bp for both ITRs.

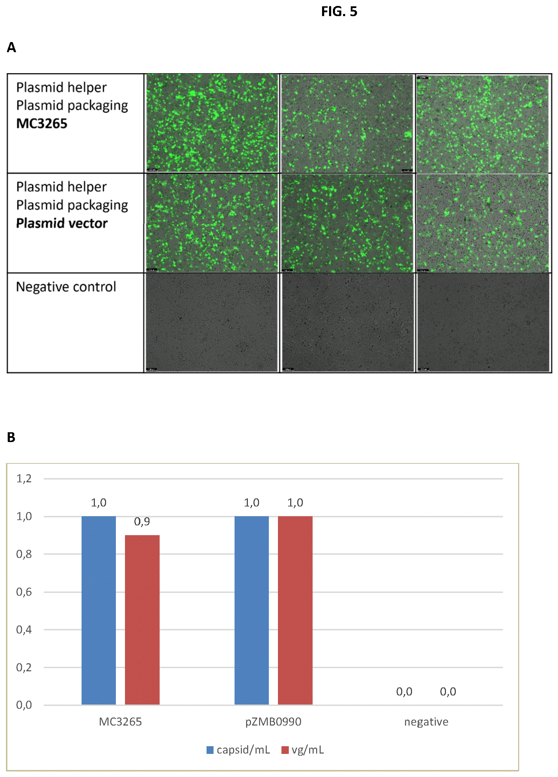

Minicircle MC3265 has already proven functional in initial application experiments (Fig. 5). In a comparative triple transfection experiment, either the initial plasmid pZMB0990 or minicircle MC3265, together with plasmids for packaging and helper functions, were transfected into the HEK293 production cell line. The transfections were carried out in 6-well plates at a concentration of 4.8×10⁵ cells/well. For each construct, 1×10¹¹ copies per well were transfected (equimolar transfection). The cells were harvested 72 hours after transfection, and GFP fluorescence was visualized. The yield of the vector genome was measured by quantitative polymerase chain reaction, and the yield of the capsid was measured by ELISA. Reporter gene expression was similar under both conditions (Fig. 5 A). The measured vector genomes and capsid yields were similar in these initial experiments, whether using the ITR plasmid with full-length ITRs or the ITR minicircle construct with full-length ITRs (Fig. 5 B).

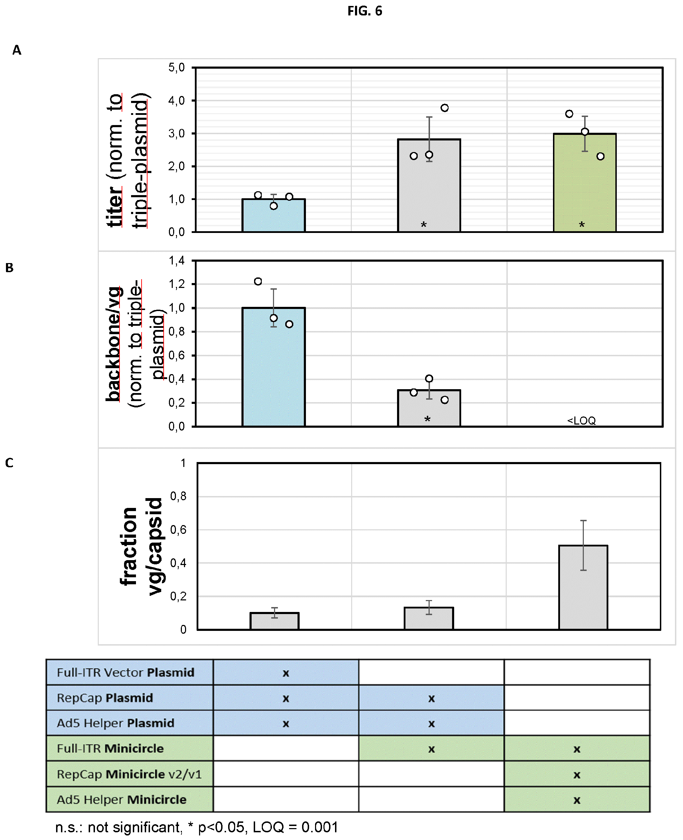

When the constructs were transfected on the basis of equal mass of total DNA instead of an equimolar basis, a significant increase in the vector genome yield was noted with the full-length ITR minicircle MC3265 compared to the full-length ITR plasmid pZMB0990. Bacterial backbone sequence packaging was significantly reduced, whereas packaging efficiency (capsid loading) showed a marked advantage for the combination of minicircle transfer, packaging and helper constructs (Fig. 6).

### EXAMPLE 6

### Cultivation of a parental plasmid comprising full-length ITRs in a fermenter

10 ng of parental plasmid PF3265PP were added to 50 µl of competent *E. coli* cells. The cells were shaken in SOC medium at 42°C for 15 minutes and then plated on LB-kanamycin plates pre-warmed to 42°C. After incubation for 16 h, colonies on the plate were counted and documented. Correct clones were identified by colony PCR. In parallel, cells were removed from the colonies and transferred into liquid culture medium (4 ml) pre-warmed to 42°C in culture tubes shaken at 42°C. After 12 h, 400 µl of culture were removed from several tubes identified as containing correct clones and demonstrating growth. Each tube was inoculated into shake flasks containing 40 ml of culture medium plus kanamycin (final concentration 75 µg/ml) as a pre-culture. The pre-culture was shaken at 42°C and 120 rpm to maintain ITRs at full-length.

After 8 h of cultivation, the flasks were checked for growth. The parental plasmid was isolated from 5 ml of culture. In-process control of the DNA in the pre-culture was performed in parallel using restriction digestion, agarose gel electrophoresis, and capillary gel electrophoresis. Only if the latter yielded a single peak for the ITR fragment could the clone be considered suitable for the inoculum. The inoculum for the main culture was selected.

A fermenter with a working volume of 30 l of culture medium was prepared. The technical parameters were 1 vvm aeration rate, an initial stirrer speed of 200 rpm, a pH of 7.0, and a temperature of 42°C.

After a total of 24 h of cultivation, including 12 h of pre-culture in tubes and 12 h of pre-culture in flasks, the cells from the selected colony present in the remaining 35 ml of culture in the shake flask were used to inoculate the fermenter by sterile injection. Batch fermentation was carried out in the absence of antibiotics. After 4 h, a sample was obtained as a pre-induction sample, and the optical density was measured. Recombination was then induced to produce the minicircle, followed by incubation at 42°C for 1 h. The bacterial culture was harvested while maintaining the bioreactor temperature at 42°C and with slow mixing at 200 rpm. First, 3 l of the bacterial culture was harvested into a pre-chilled 5 l beaker and immediately distributed into six pre-chilled centrifuge bottles. The culture was centrifuged at 4°C and 7000 rpm for 6 min. The remainder of the bacterial culture was harvested similarly. The collected bacterial cells were packaged and immediately stored at -20°C. Samples were taken from the biomass for DNA extraction (recombinant product) and subsequent analysis by spectrophotometry, restriction digestion, agarose gel electrophoresis, and nanopore sequencing.

### EXAMPLE 7

### Generation of new AAV transfer constructs carrying a reporter gene and wild-type length ITRs

An AAV transfer plasmid, as initially used, contains functional but truncated ITRs that impair genome packaging and reduce vector yield. Using the wild-type length ITR containing plasmid PF3260 (pZMB0990) as a donor for the full-length inverted repeat sequences, these crucial sequence elements were taken and cloned into the transfer plasmid containing ITRs with deletions.

The status of the ITR elements was verified using Oxford Nanopore Sequencing using the native barcoding kit whereby the plasmids were linearized at a specific position previously. Altogether three plasmids were created. Two of the new ITR plasmids with the restored ITR lengths carried different backbones - one containing an ampicillin resistance and another containing a kanamycin resistance gene. The third plasmid was a completed parental plasmid whereby the full-length ITR Gene of Interest cassette was cloned in between recombination sites.

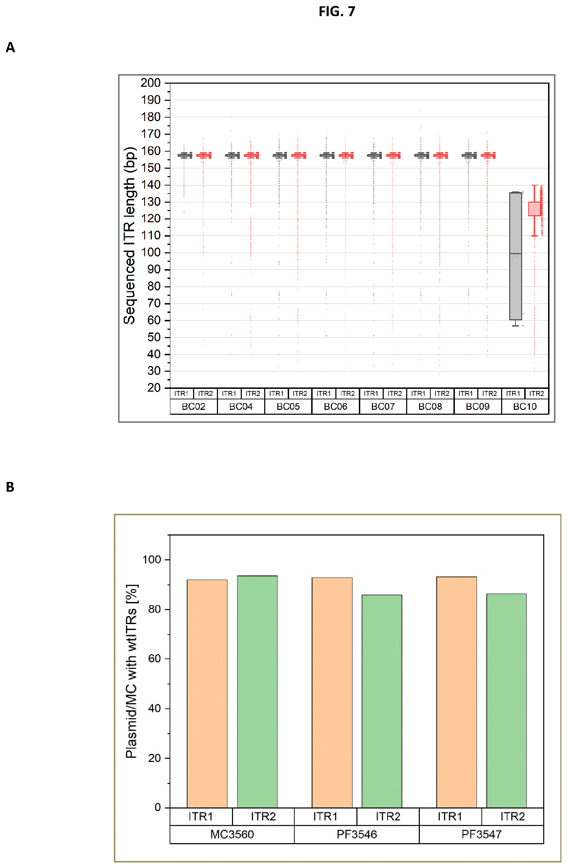

The cloning strategy took into consideration the need to separate the origin proximal ITR by at least 438 bp from the origin of replication sequence as published earlier (Radukic et al. 2024). This was to reduce the chances for this ITR to be particularly prone to degradation effects due to its proximity to the replication bubble. The difference in the measured ITR lengths and thus the extent of characterization of these repeat elements is made evident in Fig.7 . Bases flanking the ITRs were defined as reference sequences in order to differentiate between the proximal and distal ITRs and thus leading to 157 bp marked as ITRs for the reference sequence within which the 144 bp wild-type sequence was contained. The single molecule sequencing reads were aligned to the reference sequence, sorted, filtered for quality and the aligned bases were given as output in the form of measured ITR lengths for each ITR. It can be seen in Fig.7 that the newly cloned constructs with the ITRs containing restored lengths all have a high degree of homogeneity and their measured lengths fall very closely to each other around the expected value. The original plasmid contained functional but truncated ITRs and could obviously never match the measured lengths of the new constructs. The effect of the length restoration is evident in the difference of the measured lengths in this diagram.

Transformation was into chemically competent cells of a strain PF37 which is of sbcC knockout in addition to carrying the genetic markers recA⁻ and endA⁻. The gene of interest in the AAV transfer plasmid was an eGFP reporter along with a hygromycin selection marker. The strain was cultivated at 42°C.

Thus, a form of ITR repair is possible using such plasmids.

The parental plasmid described in Fig.7A (BC02) carried the designated number PF3560PP. Upon cultivation of this plasmid in the sbcC knockout strain PF37 at 42°C and the successful combination with the Minicircle production process, it was possible to visualize the supercoiled Minicircle MC3560. This Minicircle was purified to remove the miniplasmid which is a by-product. MC3560 passed all subsequent Quality Control tests such as homogeneity, DNA concentration, identity etc. The final ITR length characterization in Fig.7B showed a high degree of homogeneity of these repeat sequences in the produced Minicircles. This was possible due to the combination of the cultivation conditions employed as well as the specifically suited genotype of the production strain. Again, a precursor plasmid that may be showing signs of degradation in one of the ITRs can result in a favourable change of representation of the ITR lengths when observed in the resulting minicircle. This is probably due to the selective effect during the cloning process for a minicircle combined with the conditions created that remove any disadvantages for molecules carrying full-length ITRs to replicate. With the optimal production strain carrying a deleted sbcC locus, it is possible to observe good maintenance of ITRs.

### REFERENCES

1. Andrews BJ, Proteau GA, Beatty LG, Sadowski PD. The FLP recombinase of the 2 micron circle DNA of yeast: interaction with its target sequences. Cell, 1985; 40(4):795-803.
2. Bateman JR, Lee AM, Wu C. Site-specific transformation of Drosophila via phiC31 integrase-mediated cassette exchange. Genetics, 2006; 173(2):769-777.
3. Berns KI. The Unusual Properties of the AAV Inverted Terminal Repeat. Hum Gene Ther, 2020; 31(9-10): 518-523.
4. Doherty JP, Lindeman R, Trent RJ, Graham MW, Woodcock DM. Escherichia coli host strains SURE and SRB fail to preserve a palindrome cloned in lambda phage: improved alternate host strains. Gene 1993, 124:29-35.
5. Eberl L, Kristensen CS, Givskov M, Grohmann E, Gerlitz M, Schwab H. Analysis of the multimer resolution system encoded by the parCBA operon of broad-host-range plasmid RP4. Mol Microbiol, 1994; 12(1):131-141.
6. Hauser MA, Scocca JJ. Site-specific integration of the Haemophilus influenzae bacteriophage HP1. Identification of the points of recombinational strand exchange and the limits of the host attachment site. J Biol Chem 1992; 267(10):6859-6864.
7. Landy A, Ross W. Viral integration and excision: structure of the lambda att sites. Science 1977; 197(4309):1147-1160.
8. Leong JM, Nunes-Düby S, Lesser CF, Youderian P, Susskind MM, Landy A. The phi 80 and P22 attachment sites. Primary structure and interaction with Escherichia coli integration host factor. J Biol Chem, 1985; 260(7):4468-4470.
9. Lusby E, Bohenzky R, Berns KI. Inverted terminal repetition in adeno-associated virus DNA: independence of the orientation at either end of the genome. J Virol, 1980; 34(2):402-409.
10. Mayrhofer P, Blaesen M, Schleef M, Jechlinger W. Minicircle-DNA production by site specific recombination and protein-DNA interaction chromatography. J Gene Med. 2008; 10(11):1253-1269.
11. Radukic MT, Le DT, Krassuski T, Borchert P, Leach DRF, Müller KM. Degradation and stable maintenance of adeno-associated virus Inverted Terminal Repeats in E. coli. Nucleic Acids Res, 2025; 53(2):gkae1170.
12. Remaut E, Tsao H, Fiers W. Improved plasmid vectors with a thermoinducible expression and temperature-regulated runaway replication. Gene, 1983; 22(1):103-113.
13. Rudolph B, Gebendorfer KM, Buchner J, Winter J. Evolution of Escherichia coli for growth at high temperatures. J Biol Chem, 2010; 285(25):19029-19034.
14. Samulski RJ, Berns Kl, Tan M, Muzyczka N. Cloning of adeno-associated virus into pBR322: rescue of intact virus from the recombinant plasmid in human cells. PNAS, 1982; 79(6):2077-2081.
15. Samulski RJ, Srivastava A, Berns KI, Muzyczka N. Rescue of adeno-associated virus from recombinant plasmids: gene correction within the terminal repeats of AAV. Cell, 1983; 33(1):135-143.
16. Samulski RJ, Chang LS, Shenk T. A recombinant plasmid from which an infectious adeno-associated virus genome can be excised in vitro and its use to study viral replication. J Virol, 1987; 61(10):3096-3101.
17. Schn6dt M, Schmeer M, Kracher B, Krüsemann C, Espinosa LE, Grünert A, Fuchsluger T, Rischmüller A, Schleef M, Büning H. DNA Minicircle Technology Improves Purity of Adeno-associated Viral Vector Preparations. Mol Ther Nucleic Acids, 2016; 5(8):e355.
18. Stark WM, Boocock MR, Sherratt DJ. Catalysis by site-specific recombinases. Trends Genet, 1992; 8(12):432-439.
19. Waldman AS, Haeusslein S, Wilman G. Purification and characterization of herpes simplex virus (type 1) thymidine kinase produced in Escherichia coli by a high efficiency expression plasmid utilizing a lambda PL promoter and cl857 temperature-sensitive repressor. J Biol Chem, 1983; 258(19): 11571-11575.
20. Wang XS, Ponnazhagan S, Srivastava A. Rescue and replication signals of the adeno-associated virus 2 genome. J Mol Biol, 1995; 250(5):573-580.
21. Wilmott P, Lisowski L, Alexander IE, Logan GJ. A User's Guide to the Inverted Terminal Repeats of Adeno-Associated Virus. Hum Gene Ther Meth, 2019; 30(6):206-213.
**The following items constitute embodiments of the invention:**
1. A minicircle comprising at least one inverted repeat of viral origin, wherein said inverted repeat is essentially not truncated.
2. The minicircle of item 1, wherein said inverted repeat has a sequence identity of at least 90%, at least 92%, or at least 95% compared to the corresponding wild-type inverted repeat of the virus of origin.
3. The minicircle of item 1 or item 2, wherein said inverted repeat is an inverted terminal repeat (ITR), preferably an ITR of an adeno-associated virus (AAV).
4. The minicircle of any one of the preceding items, wherein said inverted repeat is at least 100 bp, at least 110 bp, at least 120 bp, at least 130 bp, at least 140 bp, or at least 150 bp in length.
5. A composition comprising a population of minicircles according to any one of the preceding items, wherein the inverted repeats of the population of minicircles have an average sequence identity of at least 90%, at least 92%, or at least 95% to the corresponding wild-type inverted repeat of the virus of origin.
6. A method for producing a minicircle from a parental plasmid, wherein the minicircle comprises at least one inverted repeat, the method comprising the steps:
   (i) transforming *Escherichia coli (E. coli)* cells with said parental plasmid;
   (ii) continuously cultivating said *E. coli* cells at a temperature of at least 39°C, comprising propagating the parental plasmid of step (i); and
   (iii) inducing the formation of minicircles from the propagated parental plasmid of step (ii).
7. The method of item 6, wherein cultivating in step (ii) is performed at 42°C.
8. The method of item 6 or item 7, wherein cultivating in step (ii) is performed for at least 8 hours, preferably for at least 16 hours.
9. The method of any one of items 6-8, wherein step (iii) is performed for at least 30 minutes, at least 1 hour, preferably for 1-4 hours or 4-8 hours.
10. The method of item 9, the method further comprising the step: (iv) isolating the minicircles.
11. The method of any one of items 6-10, wherein said inverted repeat is essentially not truncated, preferably wherein said inverted repeat has a sequence identity of at least 90%, at least 92%, or at least 95% compared to the corresponding inverted repeat on the parental plasmid.
12. The method of any one of items 6-11, wherein the inverted repeats of the of the minicircles obtained in step (iii) have an average sequence identity of at least 90%, at least 92%, or at least 95% to the corresponding inverted repeat of the parental plasmid.
13. The method of any one of items 6-12, wherein the inverted repeat is of viral origin.
14. The method of any one of items 6-13, wherein said inverted repeat is an ITR, preferably an ITR of an AAV.
15. The method of item 14, wherein said at least one ITR comprises an ITR proximal to the origin of replication of the parental plasmid (origin proximal ITR) and an ITR distal to the origin of replication of the parental plasmid (origin distal ITR).
16. The method of any one of items 6-15, wherein said inverted repeat is at least 100 bp, at least 110 bp, at least 120 bp, at least 130 bp, at least 140 bp, or at least 150 bp in length.
17. The method of any one of items 6-16, wherein the *E. coli* cells are wild-type *E. coli* cells, preferably K12 or B derivatives of *E. coli.*
18. The method of any one of items 6-16, wherein the *E. coli* cells have the genotype sbcC⁻ or sbcD⁻.
19. The method of any one of items 6-16 or 18, wherein the *E. coli* cells comprise a chromosomal recA⁻ and/or endA⁻ mutation.
20. The method of any one of items 6-19, wherein the minicircle comprises at least one sequence encoding:
   one or more polypeptides;
   RNA; or
   DNA;
   preferably wherein the at least one sequence has therapeutic utility.
21. A method of propagating a plasmid in *E. coli* cells, wherein the plasmid comprises at least one inverted repeat, the method comprising the steps:
   (i) transforming *E. coli* cells with said plasmid;
   (ii) continuously cultivating the *E. coli* cells comprising the plasmid of step (i) in a pre-culture at a temperature of at least 39°C for at least 8 hours, preferably for at least 16 hours; and
   (iii) inoculating a main culture with E. coli cells from the pre-culture of step (ii) and cultivating the main culture at a temperature of at least 39°C.
22. The method of item 21, wherein the main culture has a volume of at least 5 l, at least 10 l, at least 20 l, at least 30 l, at least 50 l, at least 75 l, at least 100 l, at least 200 l, at least 300 l, at least 500 l, or at least 1000 l.
23. The method of item 22, wherein after 3 days of cultivation in step (ii) and step (iii), at least 80%, at least 90%, or at least 92% of the inverted repeats of the plasmids in the main culture of step (iii) are essentially not truncated.
24. The method of item 23, wherein after 3 days of cultivation in step (ii) and step (iii), the inverted repeats of the plasmids in the main culture of step (iii) have an average sequence identity of at least 90%, at least 92%, or at least 95% compared to the inverted repeat of the plasmid of step (i).
25. The method of any one of items 21-24, wherein the inverted repeat is of viral origin.
26. The method of any one of items 21-25, wherein said inverted repeat is an ITR, preferably an ITR of an AAV.
27. The method of item 26, wherein said at least one ITR comprises an ITR proximal to the origin of replication of the plasmid (origin proximal ITR) and an ITR distal to the origin of replication of the plasmid (origin distal ITR).
28. The method of any one of items 21-27, wherein said inverted repeat is at least 100 bp, at least 110 bp, at least 120 bp, at least 130 bp, at least 140 bp, or at least 150 bp in length.
29. The method of any one of items 21-28, wherein the *E. coli* cells are wild-type *E. coli* cells, preferably K12 or B derivatives of *E. coli.*
30. The method of any one of items 21-28, wherein the *E. coli* cells have the genotype sbcC⁻ or sbcD⁻.
31. The method of any one of items 21-28 or 30, wherein the *E. coli* cells comprise a chromosomal recA⁻ and/or endA⁻ mutation.
32. The method of any one of items 21-31, wherein the plasmid comprises at least one sequence encoding:
   one or more polypeptides;
   RNA; or
   DNA;
   preferably wherein the at least one sequence has therapeutic utility.
33. The method of any one of items 21-32, wherein said plasmid is a parental plasmid for the production of minicircles comprising at least one inverted repeat.
34. The method of items 33, wherein the method further comprises the step:
   (iv) inducing the formation of minicircles from said parental plasmid in the main culture of step (iii);
   wherein step (iv) is performed for at least 30 minutes, at least 1 hour, preferably for 1-4 hours or 4-8 hours.
35. The method of items 34, the method further comprising the step:
   (iv) isolating the minicircles.

## Claims

1. A minicircle comprising at least one inverted repeat of viral origin, wherein said inverted repeat is essentially not truncated.

2. The minicircle of claim 1, wherein said inverted repeat has a sequence identity of at least 90%, at least 92%, or at least 95% compared to the corresponding wild-type inverted repeat of the virus of origin.

3. The minicircle of claim 1 or claim 2, wherein said inverted repeat is an inverted terminal repeat (ITR), preferably an ITR of an adeno-associated virus (AAV).

4. The minicircle of any one of the preceding claims, wherein said inverted repeat is at least 100 bp, at least 110 bp, at least 120 bp, at least 130 bp, at least 140 bp, or at least 150 bp in length.

5. A composition comprising a population of minicircles according to any one of the preceding claims, wherein the inverted repeats of the population of minicircles have an average sequence identity of at least 90%, at least 92%, or at least 95% to the corresponding wild-type inverted repeat of the virus of origin.

6. A method for producing a minicircle from a parental plasmid, wherein the minicircle comprises at least one inverted repeat, the method comprising the steps:
(i) transforming *Escherichia coli* (*E. coli*) cells with said parental plasmid;
(ii) continuously cultivating said *E. coli* cells at a temperature of at least 39°C, comprising propagating the parental plasmid of step (i); and
(iii) inducing the formation of minicircles from the propagated parental plasmid of step (ii), preferably wherein cultivating in step (ii) is performed at 42°C.

7. The method of claim 6, wherein cultivating in step (ii) is performed for at least 8 hours, preferably for at least 16 hours, and/or wherein step (iii) is performed for at least 30 minutes, at least 1 hour, preferably for 1-4 hours or 4-8 hours.

8. The method of claim 7, the method further comprising the step: (iv) isolating the minicircles.

9. The method of any one of claims 6-8, wherein said inverted repeat is essentially not truncated, preferably wherein said inverted repeat has a sequence identity of at least 90%, at least 92%, or at least 95% compared to the corresponding inverted repeat on the parental plasmid.

10. The method of any one of claims 6-9, wherein the inverted repeats of the of the minicircles obtained in step (iii) have an average sequence identity of at least 90%, at least 92%, or at least 95% to the corresponding inverted repeat of the parental plasmid.

11. The method of any one of claims 6-10, wherein the inverted repeat is of viral origin, preferably wherein said inverted repeat is an ITR, more preferably an ITR of an AAV.

12. The method of claim 11 or 15, wherein said at least one ITR comprises an ITR proximal to the origin of replication of the parental plasmid (origin proximal ITR) and an ITR distal to the origin of replication of the parental plasmid (origin distal ITR).

13. The method of any one of claims 6-12 or 15, wherein said inverted repeat is at least 100 bp, at least 110 bp, at least 120 bp, at least 130 bp, at least 140 bp, or at least 150 bp in length, and/or wherein the *E. coli* cells are wild-type *E. coli* cells, preferably K12 or B derivatives of *E. coli*, and/or, wherein the *E. coli* cells have the genotype sbcC⁻ or sbcD⁻, and/or wherein the *E. coli* cells comprise a chromosomal recA⁻ and/or endA⁻ mutation.

14. The method of any one of claims 6-13, wherein the minicircle comprises at least one sequence encoding:
one or more polypeptides;
RNA; or
DNA;
preferably wherein the at least one sequence has therapeutic utility.

15. A method of propagating a plasmid in *E. coli* cells, wherein the plasmid comprises at least one inverted repeat, the method comprising the steps:
(i) transforming *E. coli* cells with said plasmid;
(ii) continuously cultivating the *E. coli* cells comprising the plasmid of step (i) in a pre-culture at a temperature of at least 39°C for at least 8 hours, preferably for at least 16 hours; and
(iii) inoculating a main culture with *E.* coli cells from the pre-culture of step (ii) and cultivating the main culture at a temperature of at least 39°C, preferably wherein the inverted repeat is of viral origin, more preferably wherein said inverted repeat is an ITR, in particular an ITR of an AAV.

16. The method of claim 15, wherein the main culture has a volume of at least 5 l, at least 10 l, at least 20 l, at least 30 l, at least 50 l, at least 75 l, at least 100 l, at least 200 l, at least 300 l, at least 500 l, or at least 1000 l, and/or wherein after 3 days of cultivation in step (ii) and step (iii), at least 80%, at least 90%, or at least 92% of the inverted repeats of the plasmids in the main culture of step (iii) are essentially not truncated, preferably wherein after 3 days of cultivation in step (ii) and step (iii), the inverted repeats of the plasmids in the main culture of step (iii) have an average sequence identity of at least 90%, at least 92%, or at least 95% compared to the inverted repeat of the plasmid of step (i).

17. The method of claim 15, wherein the plasmid comprises at least one sequence encoding:
one or more polypeptides;
RNA; or
DNA;
preferably wherein the at least one sequence has therapeutic utility, and/or wherein said plasmid is a parental plasmid for the production of minicircles comprising at least one inverted repeat, and/or wherein the method further comprises the step:
(iv) inducing the formation of minicircles from said parental plasmid in the main culture of step (iii);
wherein step (iv) is performed for at least 30 minutes, at least 1 hour, preferably for 1-4 hours or 4-8 hours, preferably the method further comprising the step:
(iv) isolating the minicircles.
